# EUROPEAN PATENT SPECIFICATION

(11) **EP 0 460 752 B1**
(45) Date of publication and mention of the grant of the patent: **28.08.1996**
(21) Application number: 91201341.4
(22) Date of filing: 31.05.1991
(51) Int. Cl.: G03B 42/02, G21K 3/00, G01N 23/04, A61B 6/10

(54) **X-ray image pick-up method**
Röntgenbild-Aufnahmeverfahren
Procédé pour prendre des images par rayons X

(30) Priority: 06.06.1990 US 533841
(43) Date of publication of application: 11.12.1991
(73) Proprietor: Fujisaki, Yukio, Tosu-shi, Saga 841 (JP)
(72) Inventor: Fujisaki, Yukio, Tosu-shi, Saga 841 (JP)
(74) Representative: Kupecz, Arpad

(56) References cited:
- DE-A- 2 530 315
- US-A- 4 727 561
- US-A- 4 901 337

## Description

### BACKGROUND OF THE INVENTION

This invention relates to X-ray image pick-up method and, more particularly, to a penetration type method of picking up X-ray image with a remarkably reduced radiation dose and at a high resolution.

The X-ray image pick-up method of the kind referred to finds itself to be useful as utilized in medical diagnostic field with its ability of quickly diagnosing internal affected part of human body at an early stage.

### DESCRIPTION OF RELATED ART

For the purpose of the medical diagnosis of the internal affected part, generally, the penetration type X-ray image pick-up method has been most frequently employed. That is, X-rays radiated from an X-ray tube and penetrated through an object are directly picked up by a camera of the image intensifier type (which shall be hereinafter referred to simply as "I.I.") or taken by an X-ray film.

In attaining a proper X-ray image, in general, it becomes necessary to increase X-ray radiation dose and also to obtain a number of X-ray images of the affected part from different angles, so that there arises a problem that doctors or X-ray operators working at medical facilities are forced to have a larger radiation dose. According to a presentation of the United States Academy of Science in December, 1989, there has been found that a proportional relationship exists between the radiation dose and radiogenic lesions, and that the rate of occurrence of such radiogenic lesion as cancer at the stomach and so on is raised to be three to four times much by repeated irradiation in particular of a low level radioactive rays for relatively short period. While X-ray CT method and the like are known to be effective remarkably in real-time diagnosis of affected part, the X-ray radiation dose has to be increased in response to required time for the image pick-up.

In the X-ray image pick-up method, on the other hand, there has been a problem that a so-called beam hardening phenomenon takes place, in which, for example, the thickness of a bone is not in proportion to the blackening degree in a picked-up bone image on the X-ray film. In other words, while X-rays show a continuous energy spectrum distribution, this distribution shows a pattern which is inclined onto one side so as to be troublesome in carrying out an accurate image pick-up. The foregoing X-ray CT method has eliminated this beam hardening through a complicated computerized correction carried out temporarily, but this involves a problem that required facility becomes remarkably costly.

A measure for overcoming the problem of the beam hardening phenomenon has been suggested by the present inventor, as has been described in U.S. Patent No. 4,727,561 and U.S. Patent Application Serial No. 299,538, filed January 23, 1989, now US-A-4 933 960, in which X-rays from a single X-ray tube are made to be received by one of two sensors without penetration through the object to be measured and are also made to be received by the other sensor after being passed through a filter involving no variation in radiation absorption coefficient in response to the thickness of the object to be measured and through the object to be measured, and a highly precise analysis of the object can be realized with a ratio of the intensity of thus received rays at the two sensors obtained for effectively avoiding any influence of the beam hardening phenomenon.

A method for picking up X-rays for imaging purposes is known from DE-A-25 30 315. However, no measure is suggested to counteract the beam hardening phenomenon.

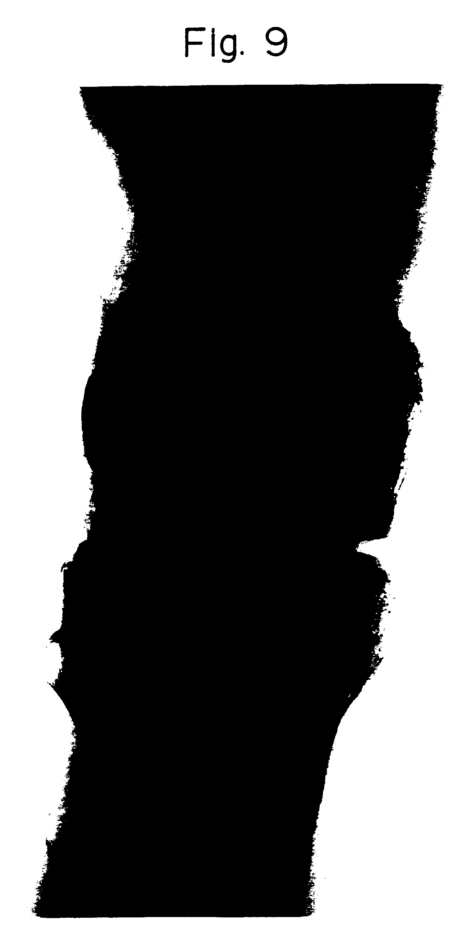

The publication of Frank Herbert Attix: "Introduction to radiological physics and radiation dosimetry", John Wiley, New York (1986) discusses in chapter 9 the well known effects of filtering on X-rays (pages 219 to 227, see in particular figure 9.10 and page 220 from the middle of the page downwards). It also discusses the fact that the beam hardening is reduced by increased filtering of the incident X-ray beam. However, no suggestion is put forward for reducing the beam hardening phenomenon in a practical medical X-ray image pick-up system.

According to the present invention this object can be realized by a method as defined in claim 1. Advantageous embodiments of this method are defined in the dependent claims.

In the present invention of the foregoing arrangement, the X-rays are irradiated upon the object with the fluctuation in the effective X-ray energy restricted to be within ± 10%, so that the beam hardening phenomenon can be effectively avoided for attaining excellent resolution, and in particular a remarkable reduction in the radiation dose can be well realized.

According to the X-ray image pick-up method of the present invention, therefore, there can be attained such effect that the X-ray image pick-up can be realized with a high resolution so as to be sufficiently contributive to the medical diagnosis and the like to render them to be accurate and reliable, and the radiation dose in particular can be remarkably reduced for remarkably reducing the radiation dose to which the patients, doctors and X-ray operators are ought to be exposed so as to effectively prevent the radiogenic lesion occurrence.

Other objects and advantages of the present invention shall be made clear in following description of the invention detailed with reference to a preferred embodiment shown in accompanying drawings.

### BRIEF DESCRIPTION OF THE DRAWINGS

FIGURE 1 is an explanatory view for an entire arrangement of elements required for carrying out the X-ray image pick-up method according to the present invention;
FIG. 2 is an X-ray spectrum distribution diagram according to the method of the present invention as represented in FIG. 1;
FIGS. 3a and 3b are X-ray spectrum distribution diagrams according to different image pick-up methods from the method of the present invention and shown in contrast to FIG. 2;
FIG. 4 is a typical diagram showing the relationship between the depth of focus of X-rays and the thickness of the object the image of which is to be picked up according to the present invention as shown in contrast to the relationship not according to the present invention;
FIG. 5 is a typical diagram showing the relationship between the depth of focus of X-rays and a gradient of the object the image of which is picked up according to the present invention as shown in contrast to that not according to the present invention;
FIG. 6 is a diagram showing the relationship between the thickness of the object and the effective energy of X-rays in the method according to the present invention;
FIG. 7 is a similar diagram showing the relationship between the thickness of the object and the effective energy of X-rays in a method not according to the present invention;
FIG. 8 is an X-ray photograph taken according to the method of the present invention; and
FIG. 9 is an X-ray photograph of the same part as FIG. 8 and taken according to a known method.

While the present invention shall now be described with reference to the embodiment shown in the accompanying drawings, it should be appreciated that the intention is not to limit the present invention only to the embodiment shown but rather to include all modifications, alterations and equivalent arrangements possible within the scope of appended claims.

### DETAILED DESCRIPTION OF THE PREFERRED EMBODIMENT

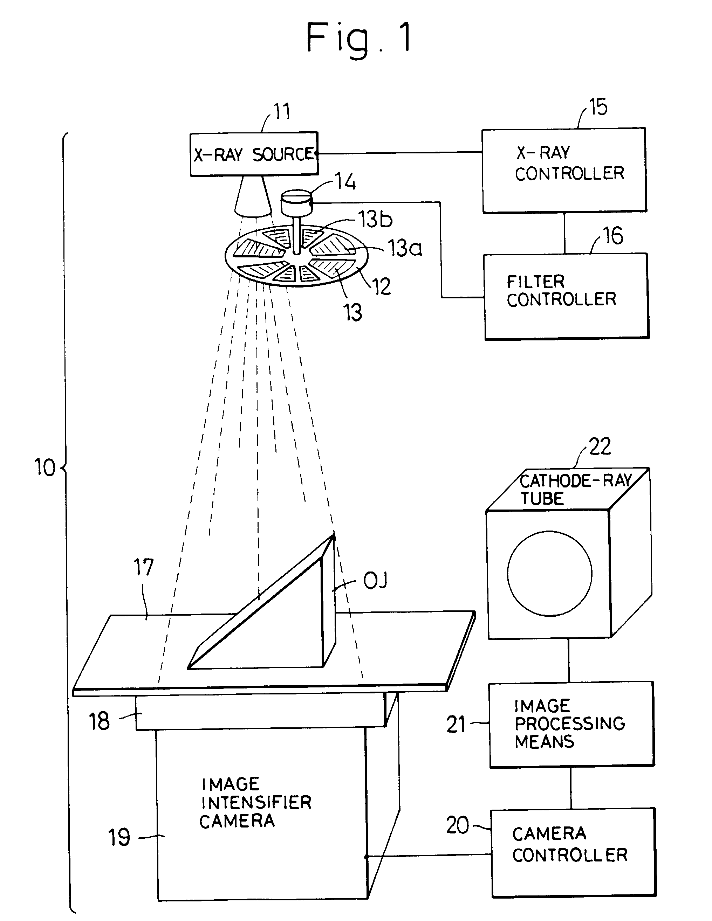

Referring here to FIG. 1 showing a system 10 employable for the X-ray image pick-up method according to the present invention, in which system 10 such X-ray source 11 as an X-ray tube is included. The source 11 is provided for radiating hard X-rays of less than 0,1 nm (1Å), and a rotary filter disk 12 is disposed below the X-ray source 11. The rotary filter disk 12 comprises a plurality of filter members 13, 13a, 13b ... 13n respectively disposed in the disk 12 as mutually spaced in circumferential direction so that, when the disk 12 is axially rotated by means of a rotary driving means 14 for a proper rotary angle, a proper one of the filter members 13, 13a, 13b ... 13n is positioned right below the X-ray source 11. These filter members 13, 13a, 13b ... 13n are made mutually different in correspondence to various spectrum distribution of the X-rays from the X-ray source 11 and eventually to various X-ray intensities of the X-ray source 11, and are so set as to cut a lower energy side of the X-rays emitted from the source 11 to restrict any fluctuation in the effective central energy of the X-rays [emitted by the source] after being penetrated through an object to be within ±10% of the effective central energy of the emitted X-rays.

The X-ray source 11 as well as the rotary driving means 14 for the rotary filter disk 12 are provided respectively to be controllable through each of X-ray controller 15 and a filter controller 16, while the both controllers 15 and 16 are mutually interlocked for indexing a corresponding one of the filter members to the effective energy of X-rays radiated. The X-rays passed through the rotary filter disk 12 are caused to be irradiated onto an object OJ placed on a table 17 for picking up X-ray image of the object, and the image is picked up by an X-ray film in a film cassette 18 disposed below the table 17.

As occasion demands, it may be also possible to have the X-rays penetrating through the object picked up by an I.I. camera 19 disposed below the table 17, which camera 19 being coupled to a camera controller 20 through which the X-ray image picked up by the I.I. camera 19 is provided to an image processing means 21, and a see-through image is provided as an output of a cathode-ray tube (CRT) 22 interlocked to the image processing means 21.

In the foregoing system 10, the X-rays are radiated by the X-ray source 11 while radiated energy of the X-rays is adjusted by the X-ray controller 15. The thus radiated X-rays are passed through one of the filter members 13, 13a, 13b ... 13n which is indexed by rotating the rotary filter disk 12 so as to have the effective energy fluctuation of the X-rays restricted to be within ±10%. The rotation in this instance of the rotary filter disk 12 is carried out through the rotary driving means 14 with the filter controller 16 in accordance with an output from the X-ray controller 15 to the X-ray source 11. The X-rays passed through the indexed filter member are caused to be irradiated onto the object for picking up its image so that the see-through image of the object OJ will be picked up by the X-ray film of the film cassette 18 in a so-called direct image pick-up process or by the I.I. camera 19 to be visualized through the camera controller 20, image processing means 21 and cathode-ray tube 22.

### EXAMPLE 1:

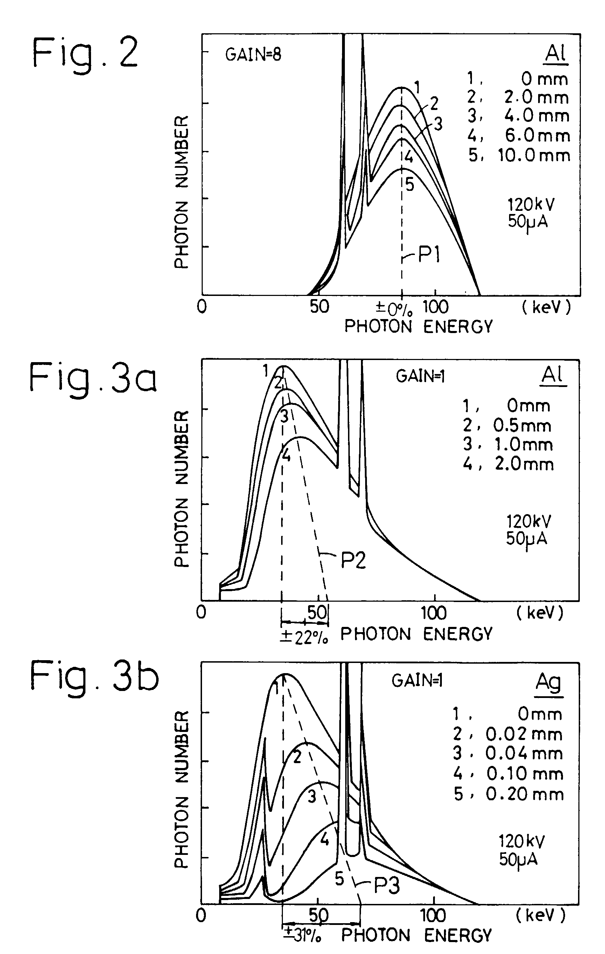

The X-ray image pick-up method according to the present invention was used, AL plates stacked so as to vary stacked thickness gradually in five steps from 0mm to 10.0mm were employed as the object for the image pick-up by the method, and the X-ray spectrum distribution was measured by means of a pure Ge semiconductor detector and a wave-height analyzer, results of which have shown that, as seen in FIG. 2, no inclination was caused to occur in the effective energy distribution, with central values of the respective energy distributions for the varying stacked thickness of the object as represented by a straight broken line P1 connecting these central values substantially aligned to be vertical. It has been found therefore that, according to the X-ray image pick-up method of the present invention, the influence of the beam hardening phenomenon could be avoided substantially and the resolution could be improved. In the present Example, the method was carried out with the tube voltage of 120kV and the tube current of 50µA for the X-ray source 11.

### COMPARATIVE EXAMPLE 1:

Except for an omission of the rotary filter disk, a substantially identical X-ray image pick-up method to that of Example 1 was employed, and the X-ray spectrum distribution was measured by means of the pure Ge semiconductor detector and wave-height analyzer with respect to the similarly stacked A1 plates with the stacked thickness varied in four steps from 0mm to 2.0mm, results of which were as in FIG. 3a. As seen therein, the effective energy distributions for the respective varying thickness have involved an inclination. That is, a straight broken line P2 connecting the center values of the respective effective energies was inclined as influenced by the beam hardening phenomenon and the fluctuation width of the effective energy has reached about ±22% to have the resolution deteriorated.

### COMPARATIVE EXAMPLE2:

Except for the omission of the rotary filter disk, a substantially identical X-ray image pick-up method to that of Example 1 was employed, the X-ray spectrum distribution was measured by means of the pure Ge semiconductor detector and wave-height analyzer with respect to a stack of Ag plates employed as the object with the stacked thickness varied in five steps from 0mm to 0.2mm, and results of such measurements have shown that, as shown in FIG. 3b by a straight broken line P3 connecting the center values of the respective effective energies and remarkably inclined, the fluctuation width of the effective energy has reached about ±31% so as to have the resolution further deteriorated.

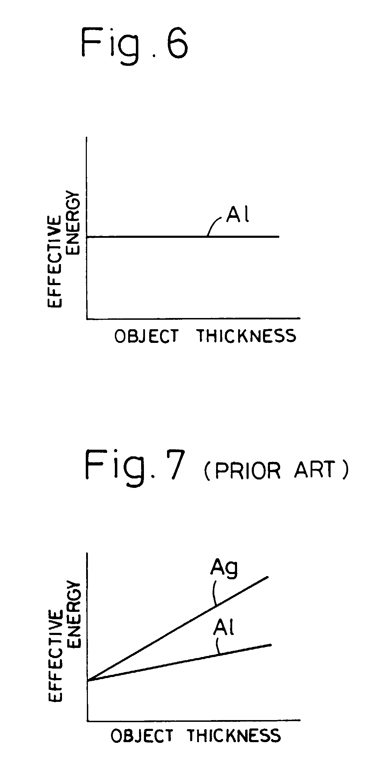

As will be clear from the foregoing Comparative Examples 1 and 2, it is considered that the object of a larger atomic number sustains a larger influence of the beam hardening phenomenon. Provided that, therefore, the atomic number of the object for the image pick-up is also taken into account as one of parameters and the indexed one of the filter members on the rotary filter disk is so selected as to be suitable for avoiding the beam hardening phenomenon, i.e., for causing no inclination in the line connecting the center values of the effective X-ray energies for the varying object thicknesses, it will be possible to obtain excellent image irrespective of the type or sort of the object. In this connection, the effective energy has been caused to fluctuate depending on the type and the atomic number of the object in the case of Comparative Examples 1 and 2, whereas in the case of Example 1 according to the present invention the fluctuation in the effective energy has been substantially restricted as shown in FIG. 6 irrespective of the type or atomic number of the object and it has been made possible to carry out the qualitative and quantitative determination of the object.

In the image pick-up method according to the present invention, too, on the other hand, the effective energy is caused to fluctuate due to variation in such conditions as source voltage, source current, room temperature, humidity and so on, but it is possible to render the effective energy fluctuation to be stationary by restricting the fluctuation to be within ±10keV when the effective energy is below 100keV and to be within ±10% when the effective energy exceeds 100keV. In other words, the X-rays immediately after being generated are subjected to the filtering so that, as represented by the straight broken line P1 in FIG. 2, the inclination of the line connecting the center values of the effective energies can be effectively restrained to render the effective energy fluctuation to be within ±10% and, more preferably, to approach ±0%.

### EXAMPLE 2:

The X-ray image pick-up method according to the present invention was used to pick up the image of an object of A1 plates sequentially stacked, and the blackening was monitored by the cathode-ray tube through the I.I. camera until the image in the central part of the stacked plates becomes invisible due to the blackening increased while peripheral outline of the topmost stacked A1 plate still remains, so that the minimum thickness of the A1 plates in which they are visible and recognizable as the object was measured. Further, a ring-shaped copper wire of a thickness of 0.6mm was placed on a stack of A1 plates as the object for the image pick-up, and the blackening of the X-ray image of the object was monitored through the I.I. camera and by the cathode-ray tube while sequentially adding the number of the A1 plates so as to build up the stack, until the copper wire ring become invisible on the monitored image due to the blackening increased by the addition of the A1 plates, whereby the maximum thickness of the stack of the A1 plates in which they are visible and recognizable as the object was measured. In the present Example, the image pick-up method was carried out with the tube voltage of 80kV and the tube current of 1mA for the X-ray cource.

### COMPARATIVE EXAMPLE 3:

Except for the omission of the use of the rotary filter disk, the same image pick-up method as in Example 2 was employed, and the minimum thickness of the stack of the A1 plates as well as its maximum thickness were measured in the same manner as in Example 2.

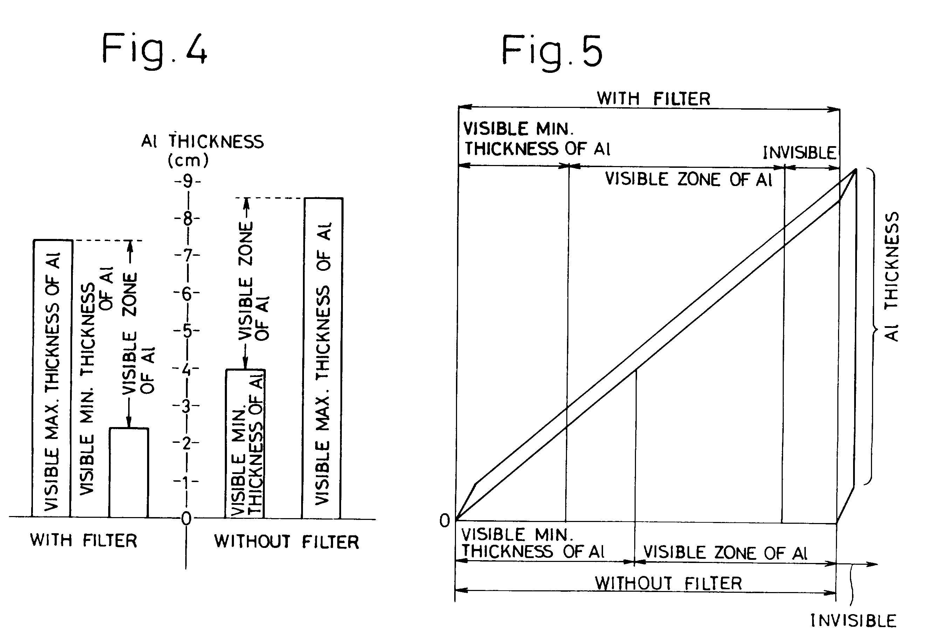

The relationship between the stacked thickness of the A1 plates and the image blakening in Example 2 and Comparative Example 3 is as shown in FIG. 4, while the extent to which the A1 plates are visible (depth of focus) and the gradient in the thickness of the stacked A1 plates are shown in FIG. 5 on the basis of FIG. 4. As a result, the radiation dose R1 required in Example 2 according to the present invention has been determined to be 13,9µC/kg/min (54mR/min)., which showing a rational decrease to be 2.7%{(R1/R2)x100} . in contrast to the radiation dose R2 of 521 µC/kg/min (2,020mR/min). required in Comparative Example 3. Further, the minimum thickness T1 of the stacked A1 plates with which the central part of the topmost A1 plate becomes invisible due to the blakening has been 2.4cm, while the same minimum thickness T2 of the stacked A1 plates with which the central part of the topmost A1 plate has become invisible in Comparative Example 3 has been 4.0cm, so that the decrease in the minimum thickness could be limited to be about 60%{(T1/T2)x100} notwithstanding the rational decrease to be 1/37 in the radiation dose. Further, in Example 2, the maximum thickness T3 of the stacked A1 plates with which the image of the copper wire ring placed on the top of the stacked A1 plates has been 7.4cm, whereas the same maximum thickness T4 in Comparative Example 3 has been 8.6cm so that the increase in the maximum thickness could be limited to be about 86%{(T3/T4)x100}. It has been found, accordingly, that the zone Z1 in which the A1 plates are visible in the X-ray image is 5.0cm(T3-T1) in Example 2 whereas the visible Zone Z2 in Comparative Example 3 is 4.6cm(T4-T2), so that the visible zone could be expanded according to Example 2 to be 109%{(Z1/Z2)x100} in comparison with Comparative Example 3.

### EXAMPLES 3-7:

The radiation dose and the visible zone of the object in the X-ray image of the object were measured in the same manner as in Example 2 but with the object for the image pick-up and the tube voltage varied as listed in a following Table 1, in which the filter members were also varied suitably as shown in the following Table 1.

### COMPARATIVE EXAMPLES 4-8:

With the object for the image pick-up and the tube voltage varied as also listed in the following Table 1, the radiation dose and the visible zone of the object were measured in the same manner as in Comparative Example 3.

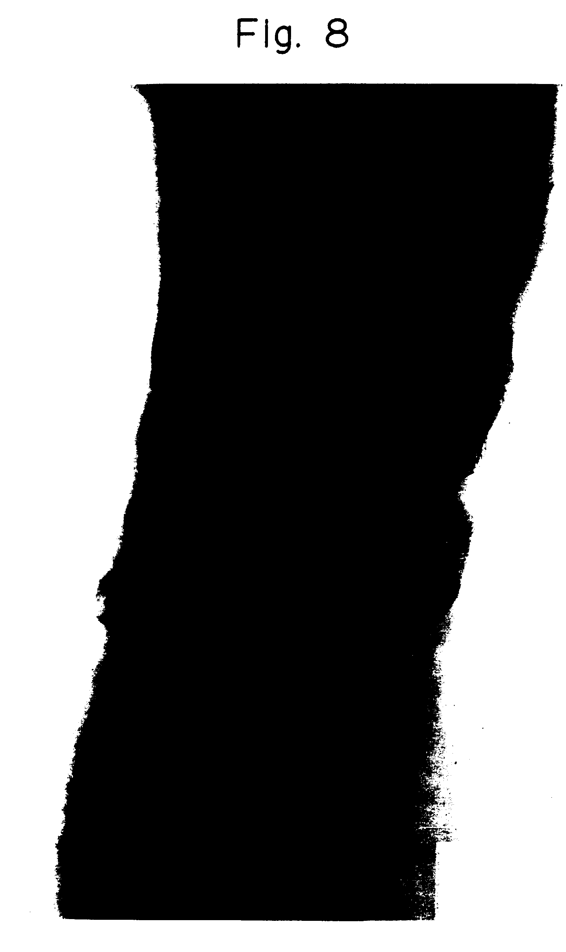

As the results of these Examples 3-7 and Comparative Examples 4-8, it has been found that, in the case when the measurement has been obtained through the X-ray image pick-up method according to the present invention, the radiation dose required for the image pick-up can be drastically reduced so as to be 1/8 to 1/25, and eventually the radiation dose to which the patients, doctors and operators must be exposed can be minimized and a satisfactory resolution can be attained, the method will be thereby made extremely useful when applied to the medical diagnoses, the resolution with respect to the object can be remarkably improved, and the visible range made larger allows a high quality X-ray image to be reliably provided. As will be clear when the X-ray photograph as in FIG. 8 for a knee picked up by the X-ray image pick-up method according to the present invention (a tube voltage of 65kV and a tube current of 100mA) is compared with the other X-ray photograph as in FIG. 9 of the same knee taken by a known method (a tube voltage of 53kV and a tube current of 100mA), it is possible to obtain the X-ray photograph in higher quality than in the case of the known method.

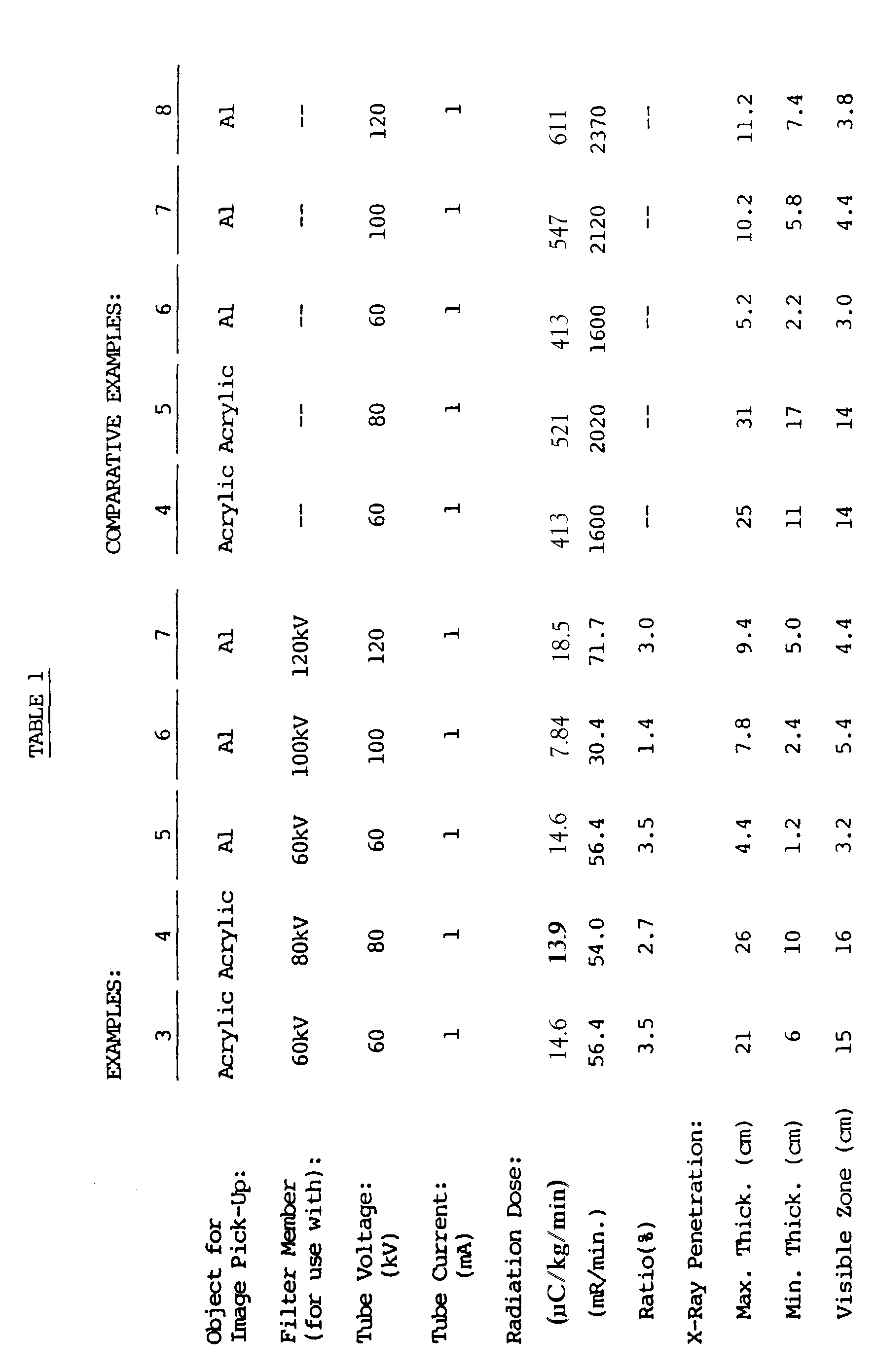

Further, the X-ray image pick-up carried out according to the method of the present invention allows an extremely high quality X-ray image to be obtained with any X-ray film available in the market, even when the tube voltage under the ordinary conditions for the image pick-up is increased by 10 to 20%, while attaining the remarkable reduction in the radiation dose.

### EXAMPLES 8 & 9:

X-ray images were obtained with an X-ray film available in the market, with the tube voltages varied as increased by 20% under the ordinary image pick-up conditions as shown in a following Table 2, and thereby obtained X-ray images could be of a superior resolution than that shown in FIG. 8. In Table 2, the radiation dose for the respective Examples is shown in comparison with the radiation dose under the ordinary image pick-up conditions.

### EXAMPLE 10:

An X-ray image was obtained in the same manner as in Examples 8 and 9, with the tube voltages increased by 10% and 20% with respect to the tube voltage under the ordinary image pick-up conditions. In Table 2, the radiation dose for the respective tube voltages is shown in comparison with that under the ordinary image pick-up conditions.

### COMPARATIVE EXAMPLES 9, 10 & 11:

Except for an omission of the use of the rotary filter disk, X-ray images were obtained in the same manner as in the above Examples 8, 9 and 10, the radiation doses in which were as shown in Table 2.

Throughout these Examples 8-10 and Comparative Examples 9-11, the tube current was made constantly 100mA. In Table 2, further, the ratio of the radiation dose for the respective Examples 8-10 is shown with respect to that of the respective Comparative Examples made 100.

As will be clear from the results in Table 2 of Examples 8-10 and Comparative Examples 9-11, the radiation dose has been drastically reduced to be 1/4 to 1/11 notwithstanding the increments in the tube voltage by 10% or 20%.

It will be appreciated that, according to the present invention, in general, the lower energy side of the X-rays, that is, the energy below 50keV in FIG. 2 is cut by the filter so that the line connecting the center values of the respective effective energies comes closer to a vertical line, whereby the radiation dose of the lower energy level than that in known method can be cut effectively, the rate of occurrence of the radiogenic lesions considered to be caused by the irradiation of low energy X-rays can be thereby lowered, and the human bodies can be effectively prevented from being injured by the X-rays.

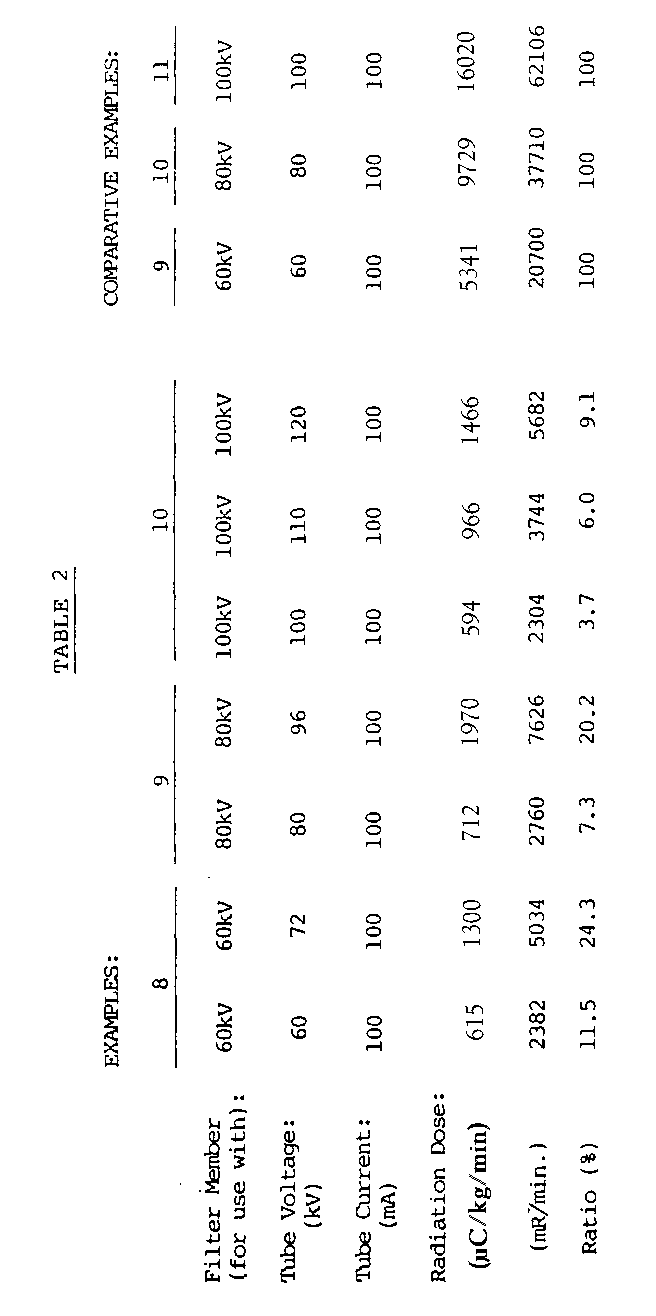

## Claims

1. A method of reducing the beam hardening phenomenon in a medical X-ray image pick-up system comprising an X-ray source (11), a filter (13) arranged in the path of the X-rays of the X-ray source and an image pick-up means (18-22) for generating a high resolution X-ray image of an object, the method comprising the steps of:
a) providing a homogeneous reference-object (OJ) of a gradually increasing thickness from substantially 0 to a predetermined maximum value and a set of filters (13, 13a, 13b, ..., 13n);
b) passing the emitted X-rays through a selected one of said filters (13, 13a, 13b, ..., 13n) for cutting-off the lower energy side portion of the energy distribution of the emitted X-rays;
c) determining the effective central energy of the energy distribution of the X-rays passed through the filter (13) and the reference-object (OJ) for each thickness portion of the reference-object (OJ);
d) comparing the effective central energies of the energy distributions obtained for the different thickness portions of the reference-object (OJ) in order to determine the differences between the effective central energies corresponding to the varying object thicknesses;
e) repeating steps b) to d) in order to select a particular filter from the set of filters (13) for which the difference between the effective central energies for the whole range of reference-object thicknesses is within about 10% of the value of the effective central energy for the lowest reference-object thickness; and
f) using the particular one of the filters (13) so selected for generating said X-ray image.

2. The method of claim 1, wherein said image pick-up means (18-22) comprises an image intensifier camera (19), image processing means (21) and a cathode-ray tube (22).

3. The method of claim 1, wherein said image pick-up means comprises a film cassette.

4. The method of claim 1, wherein the effective energy of the X-rays of lower energy level is cut as passed through said filter (13, 13a, 13b, ..., 13n).

## Patentansprüche

1. Verfahren zum Reduzieren des Strahlhärtungsphänomens in einem medizinischen Röntgenstrahlbildaufnahmesystem mit einer Röntgenstrahlquelle (11), einem im Pfad der Röntgenstrahlen von der Röntgenstrahlquelle angeordneten Filter (13) und einer Bildaufnahmeeinrichtung (18-22) zur Erzeugung eines Hochauflösungsröntgenstrahlbildes eines Objekts, wobei das Verfahren folgende Schritte aufweist:
a) Bereitstellen eines homogenen Referenzobjekts (OJ) mit allmählich von im wesentlichen 0 bis zu einem vorbestimmten Maximalwert ansteigender Dicke und eines Satzes von Filtern (13, 13a, 13b, ..., 13n),
b) Hindurchgehen der emittierten Röntgenstrahlen durch ein ausgewähltes der Filter (13, 13a, 13b, ..., 13n) zum Abschneiden des Niederenergieseitenteiles der Energieverteilung der emittierten Röntgenstrahlen,
c) Bestimmen der effektiven Zentralenergie der Energieverteilung der durch das Filter (13) und das Referenzobjekt (OJ) hindurchgegangenen Röntgenstrahlen für jeden Dicketeil des Referenzobjekts (OJ),
d) Vergleichen der für die verschiedenen Dicketeile des Referenzobjekts (OJ) erhaltenen effektiven Zentralenergien der Energieverteilungen, um die Unterschiede zwischen den den variierenden Objektdicken entsprechenden effektiven Zentralenergien zu bestimmen,
e) Wiederholen der Schritte b) bis d), um aus dem Satz von Filtern (13) ein besonderes Filter auszuwählen, für das der Unterschied zwischen den effektiven Zentralenergien für den gesamten Bereich der Referenzobjektdicke innerhalb von ungefähr 10% des Wertes der effektiven Zentralenergie für die kleinste Referenzobjektdicke liegt, und
f) Benutzen des so ausgewählten besonderen der Filter (13) zum Erzeugen des Röntgenbildes.

2. Verfahren nach Anspruch 1, worin die Bildaufnahmeeinrichtung (18-22) eine Bildverstärkerkamera (19), Bildverarbeitungseinrichtungen (21) und eine Kathodenstrahlröhre (22) aufweist.

3. Verfahren nach Anspruch 1, worin die Bildaufnahmeeinrichtung eine Filmkassette aufweist.

4. Verfahren nach Anspruch 1, worin die effektive Energie der Röntgenstrahlen niedrigeren Energieniveaus beim Hindurchgehen durch das Filter (13, 13a, 13b, ..., 13n) herabgesetzt wird.

## Revendications

1. Procédé de réduction du phénomène de durcissement d'un faisceau dans un système de prélèvement d'images à rayons X médical comprenant une source de rayons X (11), un filtre (13) agencé dans le trajet des rayons X de la source de rayons X et des moyens de prélèvement d'images (18-22) pour engendrer une image à rayons X à résolution élevée d'un objet, le procédé comprenant les étapes de :
a) fournir un objet-référence homogène (OJ) d'une épaisseur croissante graduellement d'à peu près 0 à une valeur maximale prédéterminée et un jeu de filtres (13,13a,13b...,13n) ;
b) faire passer les rayons X émis à travers l'un sélectionné parmi lesdits filtres (13,13a,13b ..., 13n) pour couper la portion latérale d'énergie basse de la répartition d'énergie des rayons X émis ;
c) déterminer l'énergie centrale effective de la répartition d'énergie des rayons X passés à travers le filtre (13) et l'objet-référence (OJ) pour chacune des portions d'épaisseur de l'objet-référence (OJ) ;
d) comparer les énergies centrales effectives des répartitions d'énergie obtenues pour les différentes portions d'épaisseur de l'objet-référence (OJ), de manière à déterminer les différences entre les énergies centrales effectives correspondant aux épaisseurs d'objet variables;
e) répéter les étapes b) à d) de manière à sélectionner un filtre particulier dans le jeu de filtres (13) pour lequel la différence entre les énergies centrales effectives pour la totalité de la gamme des épaisseurs d'objet-référence est dans à peu près 10% de la valeur de l'énergie centrale effective pour l'épaisseur d'objet-référence la plus faible ; et
f) utiliser le filtre particulier des filtres (13) ainsi choisi pour engendrer ladite image à rayons X.

2. Procédé selon la revendication 1, dans lequel lesdits moyens de prélèvement d'images (18-22) comprennent une caméra à intensification d'images (19), des moyens de traitement d'image (21) et un tube à rayons cathodiques (22).

3. Procédé selon la revendication 1, dans lequel lesdits moyens de prélèvement d'images comprennent une cassette de film.

4. Procédé selon la revendication 1, dans lequel l'énergie effective des rayons X de niveau d'énergie faible est coupée lorsqu'ils passent à travers ledit filtre (13,13a,13b,... 13n).
